# EUROPEAN PATENT APPLICATION

(11) **EP 4 497 412 A1**
(43) Date of publication of application: **29.01.2025**
(21) Application number: 23188322.4
(22) Date of filing: 28.07.2023
(51) Int. Cl.: A61F 2/07, A61F 2/00, A61F 2/06

(54) **AORTIC IMPLANT WITH PROXIMAL ANCHORING LAYER**

(71) Applicant: Medtronic Trading NL B.V., 5616 VB Eindhoven (NL); Sofradim Production, 01600 Trévoux (FR)
(72) Inventor: BAYON, Yves, Trevoux (FR); LEFRANC, Oliever, Trevoux (FR); SIMONS, Damien, Trevoux (FR); OVEREEM, Simon Pieter, Eindhoven (NL); VAN HELVOIRT, Job, Maastricht (NL)
(74) Representative: Zimmermann & Partner Patentanwälte mbB

(57) **Abstract**

An endovascular implant includes a proximal end (38), a distal end (39), and a body (37) extending between the proximal and distal ends. The body includes outer and inner surfaces. The outer surface of the body includes an outer surface landing region (32) proximate at least one of the proximal and distal ends of the body. The outer surface landing region is configured to align with one or more landing walls (34) of one or more vessels. The body further includes an outer surface aneurysmal region (43) configured to at least partially span one or more aneurysms of the one or more vessels. The endovascular implant also includes an anchoring layer (36) applied to the outer surface landing region. The anchoring layer has anchors configured to anchor the endovascular implant to the one or more landing walls of the one or more vessels. The outer surface aneurysmal region does not include the anchoring layer.

## Description

### TECHNICAL FIELD

The present disclosure relates to an aortic implant (e.g., a stent graft) with a proximal anchoring layer. In one or more aspects, the proximal anchoring layer is configured to anchor the aortic implant to a landing zone within the aorta and to resist migration of the aortic implant.

### BACKGROUND

Endovascular stent grafts may be used to treat abdominal aortic aneurysms. The stent graft is an implantable device made of a tube-shaped surgical graft material and an expanding (e.g., self-expanding) stent frame. The stent graft may be a straight tube or a bifurcated tube. The stent graft may be formed of a woven textile supported by a stent. The stent may be formed of nitinol. The stent graft is placed inside a patient's vasculature (e.g., blood vessel) to bridge a diseased blood vessel segment (e.g., an aneurismal, dissected, or torn blood vessel segment), thereby excluding hemodynamic pressures of blood flow from the diseased blood vessel segment.

Endovascular stent grafts may be used to treat abdominal aortic aneurysms and/or thoracic aortic aneurysms. During the endovascular procedure, the delivery and positioning of the stent graft may be guided by x-ray imaging. When properly positioned, the stent graft is anchored on a healthy segment of the aortic vessel wall, optionally with barbs on an anchoring stent. These barbs are configured to resist the migration of the stent graft. However, migration and permeation of the stent graft and microleakage may still occur, thereby increasing the risk of non-leak proof anchoring and aneurysm sac expansion.

### SUMMARY

In an embodiment, an endovascular implant (10) is disclosed. The endovascular implant includes a proximal end (38), a distal end (39), and a body (37) extending between the proximal and distal ends. The body includes outer and inner surfaces. The outer surface of the body includes an outer surface landing region (32) proximate at least one of the proximal and distal ends of the body. The outer surface landing region is configured to align with one or more landing walls (34) of one or more vessels. The body further includes an outer surface aneurysmal region (43) configured to at least partially span one or more aneurysms of the one or more vessels. The endovascular implant also includes an anchoring layer (36) applied to the outer surface landing region. The anchoring layer has anchors configured to anchor the endovascular implant to the one or more landing walls of the one or more vessels. The outer surface aneurysmal region does not include the anchoring layer.

The anchoring layer may be formed of an extracellular matrix layer (56). The extracellular matrix layer may be an artificial extracellular matrix layer. The extracellular matrix layer may be formed of an electrospun polymer material. The endovascular implant may further include an aneurysmal region layer applied to the outer surface aneurysmal region. The anchoring layer may be formed of a first material configured to promote a wound response. The aneurysmal region layer may be formed of a second material configured to promote an inflammation response (e.g., a controlled inflammatory reaction). The first material may be different than the second material.

The anchoring layer may include a prosthetic fabric (116) having first and second surfaces. The prosthetic fabric may include first barbs (120) extending from the first surface. The prosthetic fabric may include second barbs extending from the second surface. The first or second barbs may be the anchors referred to above. The first and/or second barbs may be formed from one or more bioresorbable polymers. The first and/or second barbs are coated with one or more natural biopolymers.

The anchoring layer may include a gripping polymeric material layer (150, 200). The gripping polymeric material layer may include a base portion (152, 202) and protrusions (156, 208) extending from the base portion. The protrusions may be the anchors. The base portion may include first and second surfaces. The protrusions may extend from the first surface. The second surface may be adhesively connected to the outer surface landing region. The protrusions may form a matrix of protrusions equally or randomly spaced from each other.

Further disclosed herein is an endovascular implant that includes a proximal end (38), a distal end (39), and a body (37) extending between the proximal and distal ends, wherein the body includes outer and inner surfaces, wherein the outer surface of the body includes an outer surface landing region (32) proximate at least one of the proximal and distal ends of the body, wherein the outer surface landing region is configured to align with one or more landing walls (34) of one or more vessels, wherein the body further includes an outer surface aneurysmal region (43) configured to at least partially span one or more aneurysms of the one or more vessels, wherein the endovascular implant also includes an anchoring layer (36) applied to the outer surface landing region, wherein the anchoring layer has anchors configured to anchor the endovascular implant to the one or more landing walls of the one or more vessels, and wherein the outer surface aneurysmal region does not include the anchoring layer.

### BRIEF DESCRIPTION OF THE DRAWINGS

Figure 1 is a partial cross section view of an abdominal aorta depicting a side view of a stent graft extending within the abdominal aorta and a first common iliac artery (CIA) and a second CIA.
Figure 2A is a magnified, cross section view of the graft material of a stent graft at a magnification of 500.
Figure 2B is a magnified view of the extracellular matrix layer of Figure 2A.
Figure 3A is a side view of a stent graft depicting application of gripping fabric to an outer end region of the stent graft.
Figure 3B depicts a magnified view of the gripping fabric shown in Figure 3A.
Figure 4A depicts a magnified view of a first polymeric gripping material at a magnification level of 60 µm.
Figure 4B depicts a magnified view of a second polymeric gripping material at a magnification level of 100 µm.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical applications. Various combinations and modifications of the features consistent with the teachings of this disclosure, however, could be desired for particular applications or implementations.

Directional terms used herein are made with reference to the views and orientations shown in the exemplary figures. A central axis is shown in the figures and described below. Terms such as "outer" and "inner" are relative to the central axis. For example, an "outer" surface means that the surfaces faces away from the central axis, or is outboard of another "inner" surface. Terms such as "radial," "diameter," "circumference," etc. also are relative to the central axis. The terms "front," "rear," "upper" and "lower" designate directions in the drawings to which reference is made.

Unless otherwise indicated, for the delivery system the terms "distal" and "proximal" are used in the following description with respect to a position or direction relative to a treating clinician. "Distal" and "distally" are positions distant from or in a direction away from the clinician, and "proximal" and "proximally" are positions near or in a direction toward the clinician. For the stent-graft prosthesis, "proximal" is the portion nearer the heart by way of blood flow path while "distal" is the portion of the stent-graft further from the heart by way of blood flow path.

The following detailed description is merely exemplary in nature and is not intended to limit the invention or the application and uses of the invention. Although the description is in the context of treatment of blood vessels such as the aorta, coronary, carotid, and renal arteries, the invention may also be used in any other body passageways (e.g., aortic valves, heart ventricles, and heart walls) where it is deemed useful.

Barbs provided by an anchoring stent are configured to anchor a stent graft to a healthy segment of an aortic vessel adjacent an aneurysm. These barbs resist migration of the stent graft after deployment within the aorta. Stent grafts may also not include barbs, instead relying on radial force from self-expanding stent rings. However, in certain cases, migration of the stent graft may occur, thereby increasing the risks of an endoleak. An endoleak (e.g., a type 1 endoleak) may result in re-pressurization of the aortic aneurysm sac, thereby increasing the risk of aneurysm sac expansion, which may lead to the rupture of the aortic vessel. Insufficient sealing of the implant (e.g., stent graft) may also cause microleakage, which may impede the regression of an aneurysm sac over time. This may cause type 2 endoleaks, thereby potentially compromising the success of an initial aortic aneurysm treatment.

Stent graft migration may result from an insufficient anchoring force of the stent graft and/or the lack of adequate tissue integration into the proximal segment of the aortic vessel used as the landing zone for the stent graft. Therefore, what is needed is a stent graft having increased initial proximal anchoring force and that integrates with newly formed tissues in the landing zone by accelerating tissue ingrowth. One or more embodiments include a stent graft having an anchoring layer applied to an outer surface of the stent graft to decrease migration and to enhance tissue ingrowth, thereby providing enhanced integration between the vessel and the stent graft over time.

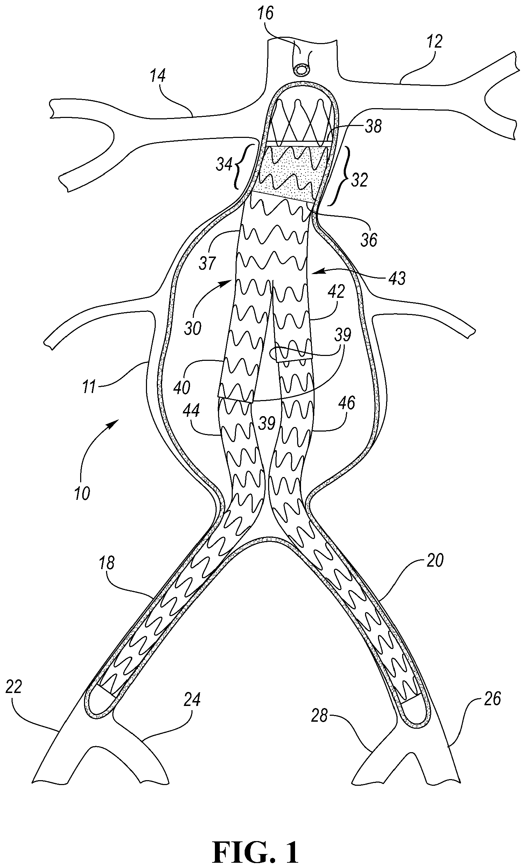

Figure 1 is a partial cross section view of abdominal aorta 10 with aneurysm 11 in the infrarenal region of abdominal aorta 10. A proximal region of abdominal aorta 10 branches into left renal artery 12, right renal artery 14, and superior mesenteric artery (SMA) 16. A distal region of abdominal aorta 10 branches into first common iliac artery (CIA) 18 and second CIA 20. First CIA 18 branches into first external iliac artery (EIA) 22 and first internal iliac artery (IIA) 24. Second CIA 20 branches into second EIA 26 and second IIA 28.

Figure 1 also depicts a side view of stent graft 30 shown in an expanded, deployed configuration where proximal region 32 aligns with landing wall 34 of abdominal aorta 10. Stent graft 30 includes body 37 extending between proximal end 38 and distal end 39. Landing wall 34 extends between left renal artery 12 and right renal artery 14 and aneurysm 11. Anchoring layer 36 is applied to the outer surface of stent graft 30. As shown in Figure 1, anchoring layer 36 is disposed within outer surface landing region 32 and may be spaced apart from proximal end 38 of stent graft 30. As shown in Figure 1, anchoring layer 36 extends a portion of the length of landing zone 34 of abdominal aorta 10. In another embodiment, anchoring layer 36 extends the entire length of landing wall 34. In one or more embodiments, anchoring layer 36 does not extend into any portion of stent graft 30 spanning aneurysm 11. Anchoring layer 36 may improve the grip and/or anchoring of stent graft 30 to the vessel wall, particularly in a proximal end region where the graft contacts the vessel wall and the two regions overlap. While Figure 1 depicts a bifurcated stent graft, one or more embodiments may be applied to straight, tubular stent grafts, branched stent grafts, fenestrated stent grafts, and other aortic implants. Also, while an abdominal aneurysm stent graft is shown, the anchoring layer 36 may be applied to stent grafts used in any portion of the aorta, such as the aortic arch or descending thoracic aorta, or in any portion of the vasculature. The anchoring layer 36 may also be applied to a transcatheter heart valve, such as a transcatheter aortic valve replacement (TAVR) or a transcatheter mitral or tricuspid valve replacement (TMVR/TTVR) to improve anchoring and/or reduce leakage (e.g., paravalvular leakage (PVL)).

Anchoring layer 36 may be formed of an extracellular matrix layer (e.g., an artificial extracellular matrix layer). The artificial extracellular matrix material may be formed by electrospinning a porous scaffold of fibers (e.g., nanofibers) of a polymeric material onto an outer surface of a stent graft proximal landing region. The artificial extracellular matrix material may be formed to mimic the surface of the aortic vessel. In one or more embodiments, the artificial extracellular matrix material has a higher surface area than the graft material of a stent graft, thereby providing enhanced friction to promote cell adhesion.

The polymeric material may be a composite of a first natural polymer and a second natural polymer different than the first natural polymer. The polymeric material may be a composite of a first natural polymer and a first synthetic polymer. The first and/or second natural polymers may be selected from the group consisting of: chitosan, collagen, gelatin, and silk. The polymeric material may be formed of a first synthetic polymer and a second synthetic polymer different than the first synthetic polymer. The synthetic polymer may be selected from the group consisting of: polycaprolactone (PCL), poly-lactide acid (PLA), poly(lactic-coglycolic acid), trimethylene carbonate (TMC), or a combination thereof. The polymeric material may be formed from one or more crosslinked polymers. The polymeric material may be formed from one or more reinforced polymers with one or more inorganic materials.

The porous nanofiber electrospun scaffold may also include one or more biomolecules and/or biomacromolecules to enhance bioactive cues and/or to enhance the scaffolds for the cells. The biomolecules and/or biomacromolecules may have a size of any of the following values or in a range of any two of the following values: 800, 825, 850, 875, 900, 925, 950, 975, and 1,000 Daltons. The biomolecules and/or biomacromolecules may include nucleic acids, proteins, and/or carbohydrates, made from monomer units linked together.

The mean diameter of the electrospun fibers may be any of the following values or in a range of any two of the following values: 75, 100, 125, 150, 175, 200, 225, 250, 275, 300, 325, 350, 375, 400, 425, 450, 475, 500, 525, 550, 575, and 600 nanometers.

The porous nanofiber electrospun polymer scaffold may form a matrix including one or more fibers, one or more link proteins, and one or more space filling molecules. The one or more link proteins may be configured to promote protein functionalization. The one or more link proteins may include fibronectin, laminin, or a combination thereof. The one or more space filling molecules may include proteoglycans, glycosaminoglycans, or a combination thereof.

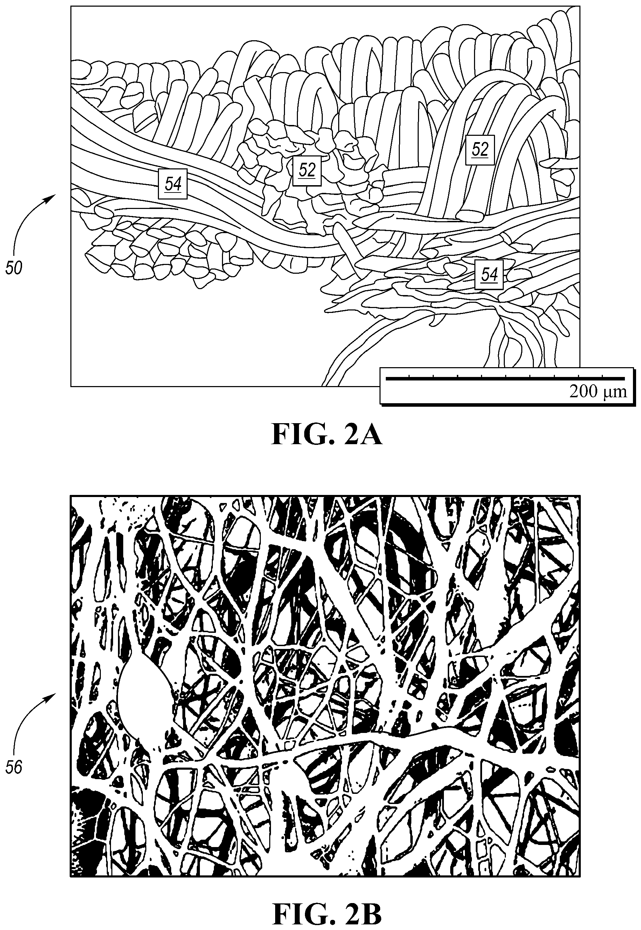

Figure 2A is a magnified, cross section view of graft material 50 in proximal region 32 of stent graft 30 at a magnification of 500. As shown in Figure 2A, the graft material 50 is formed of first fiber bundles 52 extending in a first direction and second fiber bundles 54 extending in a second direction. In one or more embodiments, first fiber bundles 52 are generally orthogonal (e.g., orthogonal within ± 5 degrees) second fiber bundles 54. The overall structure and outer surface of graft material 50 is wavy at the magnification shown in Figure 2A.

Figure 2B is a magnified view of extracellular matrix 56. In one or more embodiments, extracellular matrix 56 is an artificial extracellular matrix formed from a porous nanofiber electrospun polymer scaffold. Extracellular matrix 56 may be applied to the outer surface of graft material 50 to form an anchoring layer on the stent graft. The anchoring layer may be configured to induce and/or upregulate a positive foreign body response to the stent graft after deployment. The anchoring layer is configured to promote positive interaction between stent graft 30 and landing wall 34 of abdominal aorta 10 (e.g., including infrarenal intimal tissue as shown in Figure 1). The anchoring layer may induce fibrosis of the landing zone tissue, which, in turn, resists dilation of the landing zone region and/or proliferation/differentiation. While smooth muscle cells may be hampered by non-functioning calcium channels, the anchoring layer may return smooth muscle in a relatively high state of contractility. The anchoring layer formed of an extracellular material is configured to promote epithelial growth within the landing zone region. The anchoring layer formed of an extracellular material may also promote a positive effect on one or more types of collagens (e.g., collogen types 1 and 3). The anchoring layer formed of an extracellular material may also stimulate smooth muscle cell growth. The anchoring layer formed of an extracellular material may also promote cellular ingrowth and/or cell colonization of the graft material into the aortic wall in the landing zone region due to the open pore structure of the extracellular material. The extracellular material (e.g., the porous nanofiber electrospun polymer scaffold) has a microporous characteristic, thereby promoting improved endothelialization as compared to the material used to form a stent graft (e.g., polyester or ePTFE). The extracellular material may also be beneficial in promoting cell adhesion, proliferation, biocompatibility, and/or cell infiltration. In one or more embodiments, the anchoring layer provides a positive and beneficial wound response with the vessel wall within the landing zone region.

As shown in Figure 1, stent graft 30 includes first branch limb 40 and second branch limb 42. First branch limb extension 44 extends from first branch limb 40 into first CIA 18. Second branch limb extension 46 extends from second branch limb 42 into second CIA 20. At least a portion of the outside surfaces of stent graft 30, first branch limb extension 44, and second branch limb extension 46 spanning aneurysm 11 (e.g., an outer surface aneurysmal region 43) may be coated with an aneurysmal region layer. In one or more embodiments, at least a portion of the outer surfaces of first branch limb extension 44 and/or second branch limb extension 46 include the anchoring layer (e.g., as described above) configured to promote anchoring of first branch limb extension 44 and/or second branch limb extension 46 within first CIA 18 and second CIA 20, respectively (e.g., at the distal end or the limb extensions). Alternatively, or in addition, the branch limb extensions 44 and 46 may have the anchoring layer at their proximal ends to improve the joint strength with the branch limbs 40 and 42.

The aneurysmal region layer may be configured to promote an inflammation response from the patient, which may be beneficial in treatment of aneurysms. The aneurysmal region layer may be formed of a material configured to promote a controlled inflammatory reaction and/or acute and foreign body response. The inflammation response may promote formation for cytokines and/or macrophages (e.g., M1 and M2 macrophages). The inflammation response may promote blood clotting and/or thrombus formation and formation and/or differentiation of smooth muscle cells. The aneurysmal region layer may promote cell adhesion. The material used for the aneurysmal region layer may be different than the material used for the anchoring layer (e.g., different extracellular matrix materials). In one or more embodiments, the anchoring layer and/or aneurysmal region layer are not applied to the inner surfaces of the stent graft. In one embodiment, the anchoring layer may be applied to the inner surface of a region of the stent graft that will form an overlapping joint with an outside surface of another stent graft (e.g., it may be applied to the inner surface of the distal end of first branch limb 40 or second branch limb 42, which will receive branch limb extensions 44 and 46).

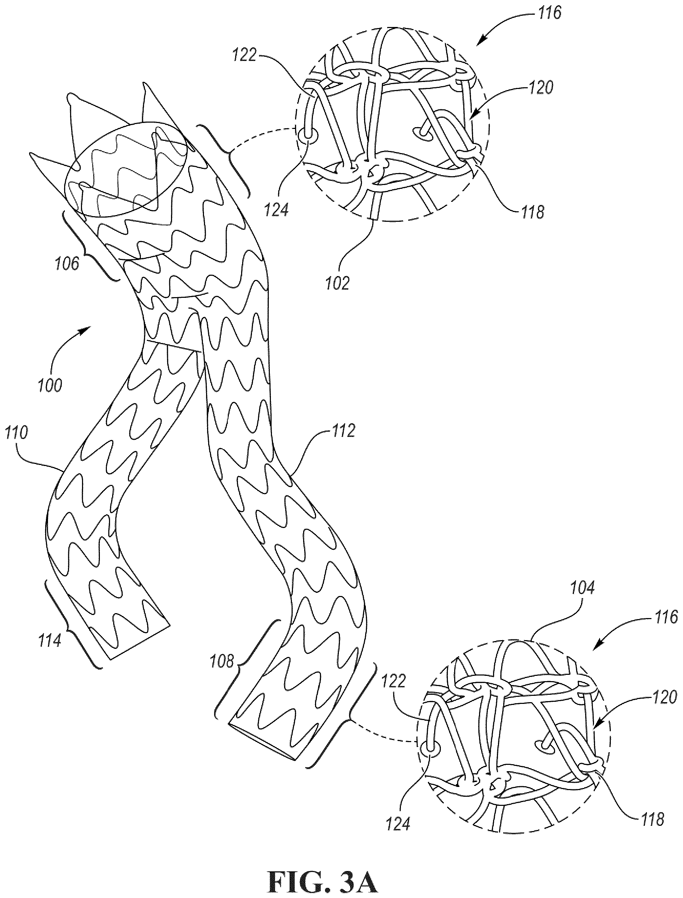

Figure 3A is a side view of stent graft 100 depicting application of anchoring layers 102 and 104 to proximal region 106 and distal region 108, respectively, of stent graft 100. Upon deployment of stent graft 100 within an abdominal aorta, proximal region 106 aligns with a landing zone region of the aortic vessel wall. Upon deployment of stent graft 100 within the abdominal aorta, distal region 106 may align with a branch vessel (e.g., first or second CIA) with a landing zone region of the branch vessel. Stent graft 100 includes first branch limb 110 and second branch limb 112. While distal region 108 is on second branch limb 112, first branch limb 110 includes distal region 114, which may align with a landing zone region of a different branch vessel and may or may not include an anchoring layer. In other embodiments, branch limbs 110 and 112 may not extend into a branch vessel, but a rather a branch limb extension may extend therefrom to a branch vessel (e.g., as shown in Fig. 1). In these embodiments, the anchoring layer may be applied to surfaces of the branch limbs and/or branch limb extensions that will form a joint together or which will contact a branch vessel (e.g., inner surface of branch limb and/or outer surface of branch limb extension where they form a joint). While Figure 3A depicts a bifurcated stent graft, one or more embodiments may be applied to straight, tubular stent grafts, branched/fenestrated stent grafts, and other aortic implants.

Anchoring layers 102 and/or 104 may be formed of a prosthetic fabric including first and second surfaces where the first and/or second surfaces may include first and/or second barbs protruding from the first and/or second surfaces, respectively. The first surface may be facing the outer surface of the stent material when applied and the second surface may be facing away from the first surface. In one embodiment, only one of the surfaces includes the barbs. In another embodiment, both surfaces include the barbs.

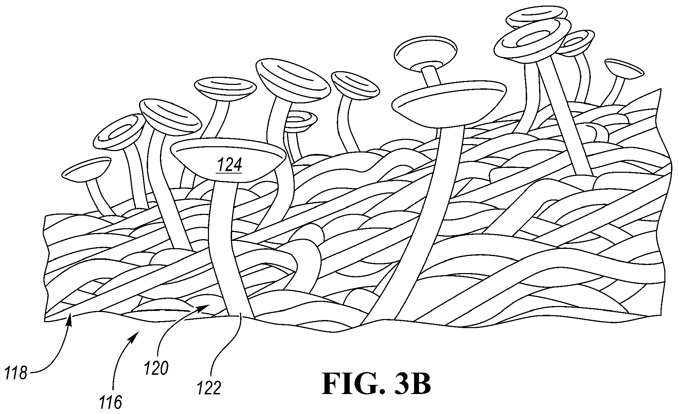

The barbs may be formed by creating a prosthetic fabric including loops. These loops may be treated (e.g., heat treated) to form first and second barbs by cleaving a single loop. Figures 3A and 3B depict prosthetic fabric 116 including base layer 118 with barbs 120 extending therefrom. Barbs 120 include neck portion 122 and grip portion 124. As shown in these figures, grip portions 124 have a bulbous profile (e.g., a diameter wider than the neck portion). Grip portions 124 may have a dimension (e.g., diameter) in the millimeter range (e.g., 1 to 10 millimeters) or the submillimeter range (e.g., 0.01 to 0.1 millimeters).

Barbs 120 are configured to anchor to the aortic wall or an iliac at the landing zone to secure a more stable fixation, thereby resisting the sliding of the stent graft. In one or more embodiments, barbs 120 are configured to mechanically stick to the aortic wall without the adjunction of chemical or biological adhesives.

Barbs, including grip portions 124, may be formed of one or more bioresorbable polymers. The bioresorbable characteristic may refer to the material having a property to be absorbed and/or degraded by tissues or washed from an implantation site or disappear in vivo after a certain time, which may vary, for example, from a few hours to a few months, thereby leaving tissue that integrates with the stent graft. Non-limiting examples of bioresorbable polymers include polylactic acid (PLA), polycaprolactone (PCL), polydioxanone (PDO), trimethylenecarbonate (TMC), polyvinyl alcohol (PVA), polyhydroxyalkanoate (PHA), polyglycolic acid (PGA), polyalkylene glycols, such as polyethylene glycol or polypropylene glycol, polysaccharides, such as starch, dextran and/or derivatives of cellulose, oxidized polysaccharides, mucopolysaccharides, copolymers thereof, and mixtures thereof. For example, the hydrophilic macromolecular additive is a polyethylene glycol having a molar mass of 4000 Daltons (PEG 4000). The bioresorbable material may include at least one polyvinyl alcohol. The bioresorbable material may include at least one glycerol. The resorption of the grips may induce a local inflammation reaction, thereby leading to the generation of new tissues that may fill the space between a stent graft and the aortic wall.

The prosthetic fabric may be applied to an outer surface of a graft material as a sheet portion (e.g., applied as a sleeve over the outer surface by sliding the sleeve over an end of the stent graft and into position within an end region of the stent graft). A dual-sided grip prosthetic fabric material may be secured to the outer surface with the grip portions of the barbs (e.g., without the use of an adhesive). A single-sided grip prosthetic fabric may be applied through adhesive between the smooth surface (e.g., not having grips) of the prosthetic fabric and the outer surface of the graft material. The adhesive may be a thermo-adhesive polymer, e.g., polycaprolactone. The graft material and the prosthetic fabric form a two-component structure configured to promote a wound response with the vessel wall within the landing zone region (e.g., for the promotion of infrarenal and iliac fixation and sealing), or smooth muscle cell differentiation.

Prosthetic fabric may mean any fabric formed of one or more biocompatible yarns, fibers, filaments, multi-filaments, or a combination thereof, using knitting, weaving, braiding or a non-woven arrangement or assembly. The arrangement of the yarns of the prosthetic fabric defines first surface and an opposing second surface. The prosthetic fabric may include barbs that protrude from at least the first surface. These barbs may protrude from the first surface substantially perpendicular to the plane of the first surface or alternatively along one more planes inclined relative to the plane of the first surface. The barbs are configured to fasten and anchor to a vessel wall within a landing zone region.

The barbs may be formed from yarns, e.g., hot-melt monofilament yarns directly resulting from an arrangement of yarns forming the fabric. The barbs may be formed from monofilament yarns made of polylactic acid.

In alternative embodiments, the barbs of the prosthetic fabric may be any hook produced from any biocompatible material, attached to the arrangement of yarns forming the fabric, whether these hooks were incorporated into said fabric during the manufacture (braiding, knitting, weaving, etc.) of the arrangement of yarns or were added thereafter.

In one or more embodiments, the barbs have the shape of a rod surmounted by a head. The average size of the head of the barbs may be any of the following values or in a range of any two of the following values: 100, 125, 150, 175, 200, 225, 250, 275, and 300 µm.

The yarns, or fibers or filaments and/or multi-filaments forming the arrangement of yarns of the fabric of one or more embodiments may be produced from any biodegradable or non-biodegradable biocompatible material, or combination thereof. The biodegradable material suitable for the yarns of the fabric may be chosen from polylactic acid (PLA), polyglycolic acid (PGA), oxidized cellulose, polycaprolactone (PCL), polydioxanone (PDO), trimethylene carbonate (TMC), polyvinyl alcohol (PVA), polyhydroxy-alkanoates (PHAs), polyamides, polyethers, copolymers of these compounds and mixtures thereof. The non-biodegradable materials suitable for the yarns of the fabric may be chosen from polyethylene terephthalate (PET), polyamides, aramids, expanded polytetrafluoroethylene, polyurethane, polyvinylidene difluoride (PVDF), polybutylesters, polyetheretherketone (PEEK), polyolefins (such as polyethylene or polypropylene), copper alloys, silver alloys, platinum, medical grades of steel such as medical grade stainless steel, and combinations thereof.

In embodiments where one surface has barbs, the other surface may be at least partially covered with an impenetrable layer (e.g., a microporous layer) produced from a solution of bioresorbable material. The bioresorbable material may be formed of a natural biological polymer material (e.g., collagen, gelatin, fibrin, fibrinogen, elastin, keratin, albumin, hydroxypropyl methyl cellulose, methyl cellulose, hydroxyethyl cellulose, hydroxypropyl cellulose, carboxyethyl cellulose, carboxymethyl cellulose, and mixtures thereof).

The barbs may also be coated with one or more natural biopolymers (e.g., collagens or hyaluronic acid) to reinforce new tissue formation. Alternatively, the barbs may be treated (e.g., with plasma surface technologies) to enhance the wettability properties (e.g., increase the hydrophobic properties at the surface) and/or to impart additional anchoring properties (e.g., the grafting of acrylate moieties by plasma surface technologies).

The prosthetic fabric, including the barbs may be treated with an antimicrobial agent. Non-limiting examples of antimicrobial agents include quaternary amines (e.g., triclosan also known under the name 2,4,4'-trichloro-2'-hydroxydiphenyl ether, polyhexamethylene biguanide (PHMB), or diallyldimethylammonium chloride also known as DADMAC, chlorhexidine and its salts (e.g., chlorhexidine acetate, chlorhexidine gluconate, chlorhexidine hydrochloride and chlorhexidine sulphate), silver and its salts (e.g., silver acetate, silver benzoate, silver carbonate, silver citrate, silver iodate, silver iodide, silver lactate, silver laurate, silver nitrate, silver oxide, silver palmitate, silver protein complex and silver sulphadiazine), polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol-9, fusidic acid, cephalosporines, and combinations thereof. Antimicrobial proteins and peptides (e.g., bovine lactoferrin and lactoferricine B) may also be suitable as a bioactive agent according to one or more embodiments.

In embodiments where both the first and second surfaces have barbs, a warp knitting machine including a needle bed including four guide bars (e.g., first, second, third, and fourth guidebars) may be used to form the dual-sided grip prosthetic fabric material, operating together and each repeating a knitting pattern defining the evolution of the yarns.

Non-limiting examples of fabrics with barbs suitable for use in one or more embodiments include Parietex^{®} Progrip or Parietene^{®} Progrip.

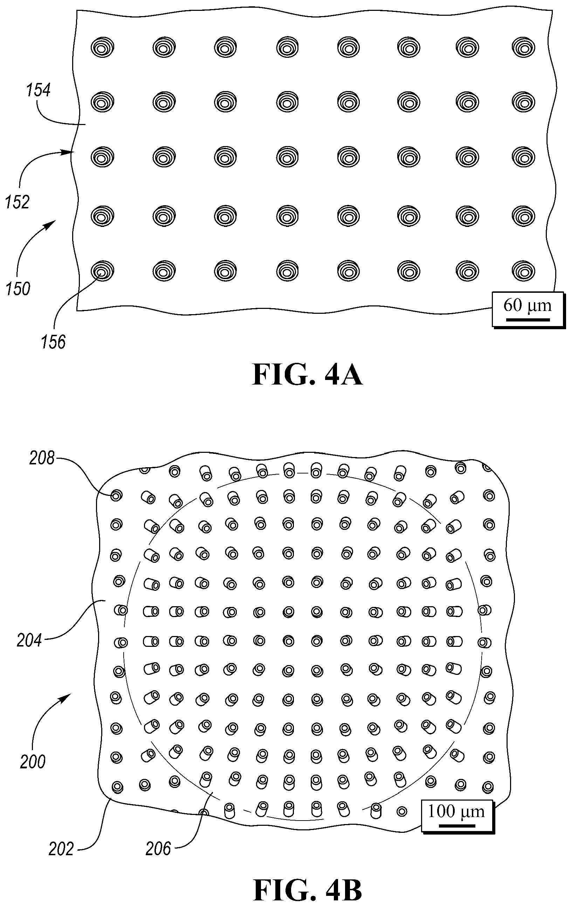

In another embodiment, a gripping polymeric material layer may be used as the anchoring layer. Figure 4A depicts a magnified view of first gripping polymeric material 150 at a magnification level of 160 µm. Figure 4B depicts a magnified view of second polymeric gripping material 200 at a magnification level of 100 µm.

As shown in Figure 4A, first gripping polymeric material 150 includes base portion 152 having first surface 154 and second surface (not shown) opposing first surface 154. Base portion 152 may be generally planar (e.g., planar within ± 5 degrees). Cylindrical shaped protrusions 156 generally orthogonally (e.g., orthogonal within ± 5 degrees) extend from base portion 152.

As shown in Figure 4B, second gripping polymeric material 200 includes base portion 202 having first surface 204 and second surface (not shown) opposing first surface 204. Base portion 202 may be generally planar except for dimples 206 extending away from the generally planar portion of base portion 202. Cylindrical shaped protrusion 208 generally orthogonally (e.g., orthogonal within ± 5 degrees) extend from base portion 202 and dimples 206. While cylindrical shaped protrusions 154 and 208 are shown with a circular profile, the cylindrical shaped protrusions of other embodiments may have a different profile (e.g., polygonal, rectangular, triangular, etc.).

As shown in Figures 4A and 4B, cylindrical shaped protrusions 154 and 208 create a matrix on base portions 152 and 202, respectively of spaced apart cylindrical shaped protrusions 154 and 208, respectively. In one or more embodiments, cylindrical shaped protrusions 154 and 208 are configured to mechanically stick to the aortic wall without the adjunction of chemical or biological adhesives. The protrusions may also be coated with one or more natural biopolymers (e.g., collagens or hyaluronic acid) to reinforce new tissue formation. Alternatively, the protrusions may be treated to enhance the wettability properties and/or to impart additional anchoring properties.

The protrusions (e.g., protrusions 154 and 208) may have a profile or dimension (e.g., diameter) of any of the following values or in a range of any two of the following values: 1, 5, 10, 15, 20, 25, 30, 35, 40, 45, 50, 55, 60, 65, 70, 75, 80, 85, 90, 95, and 100 µm. The spacing between pairs of protrusions within a matrix of protrusions may independently selected be any of the following values or in a range of any two of the following values: 1, 5, 10, 15, 20, 25, 30, 35, 40, 45, 50, 55, 60, 65, 70, 75, 80, 85, 90, 95, and 100 µm. The spacing and location of the protrusions may be regularly repeating or random. The base portion (e.g., base portion 152 or 202) may have a thickness of a thin film, e.g., any of the following values or in a range of any two of the following values: 1, 5, 10, 15, 20, 25, 30, 35, 40, 45, and 50. The gripping polymeric material may be formed of a bioresorbable material (e.g., the example materials identified above). The gripping polymeric material may be formed using a molding process (e.g., injection molding or thermal pressure molding).

The gripping polymeric material layer may be applied to an outer surface of a graft material as a sheet portion (e.g., applied as a sleeve over the other surface by sliding the sleeve over an end of the stent graft and into position within an end region of the stent graft). A dual-sided protrusion material may be secured to the outer surface with the protrusions (e.g., without the use of an adhesive). A single-sided protrusion material may be applied through adhesive between the smooth surface (e.g., not having protrusions) and the outer surface of the graft material. The adhesive may be a thermo-adhesive polymer, e.g., polycaprolactone. The graft material and the gripping polymeric material layer form a two-component structure configured to promote a wound response with the vessel wall within the landing zone region (e.g., for the promotion of infrarenal and iliac fixation and sealing).

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

Further disclosed herein is the subject-matter of the following clauses:
1. An endovascular implant (10) comprising:
   a proximal end (38);
   a distal end (39);
   a body (37) extending between the proximal and distal ends and including outer and inner surfaces, the outer surface of the body includes an outer surface landing region (32) proximate at least one of the proximal and distal ends of the body and configured to align with one or more landing walls (34) of one or more vessels, and the body includes an outer surface aneurysmal region (43) configured to at least partially span one or more aneurysms of the one or more vessels; and
   an anchoring layer (36) applied to the outer surface landing region and having anchors configured to anchor the endovascular implant to the one or more landing walls of the one or more vessels, and the outer surface aneurysmal region does not include the anchoring layer.
2. The endovascular implant of clause 1, wherein the anchoring layer includes an extracellular matrix layer (56).
3. The endovascular implant of clause 2, wherein the extracellular matrix layer is an artificial extracellular matrix layer.
4. The endovascular implant of clause 2, wherein the extracellular matrix layer is formed of an electrospun polymer material.
5. The endovascular implant of clause 1 further comprising an aneurysmal region layer applied to the outer surface aneurysmal region, the anchoring layer formed of a first material configured to promote a controlled wound response, the aneurysmal region layer formed of a second material configured to promote a controlled inflammatory reaction, and the first material is different than the second material.
6. The endovascular implant of clause 1, wherein the anchoring layer includes a prosthetic fabric (116) having first and second surfaces.
7. The endovascular implant of clause 6, wherein the prosthetic fabric includes first barbs (120) extending from the first surface.
8. The endovascular implant of clause 7, wherein the prosthetic fabric includes second barbs extending from the second surface.
9. The endovascular implant of clause 6 or 7, wherein the first or second barbs are the anchors.
10. The endovascular implant of clause 6 or 7, wherein the first and/or second barbs are formed from one or more bioresorbable polymers.
11. The endovascular implant of clause 6, 7, or 10, wherein the first and/or second barbs are coated with one or more natural biopolymers.
12. The endovascular implant of clause 1, wherein the anchoring layer includes a gripping polymeric material layer (150, 200).
13. The endovascular implant of clause 12, wherein the gripping polymeric material layer includes a base portion (152, 202) and protrusions (156, 208) extending from the base portion, the protrusions are the anchors.
14. The endovascular implant of clause 13, wherein the base portion includes first and second surfaces, the protrusions extending from the first surface, the second surface adhesively connected to the outer surface landing region.
15. The endovascular implant of clause 12 or 13, wherein the protrusions form a matrix of protrusions equally or randomly spaced from each other.

## Claims

1. An endovascular implant (10) comprising:
a proximal end (38);
a distal end (39);
a body (37) extending between the proximal and distal ends and including outer and inner surfaces, the outer surface of the body includes an outer surface landing region (32) proximate at least one of the proximal and distal ends of the body and configured to align with one or more landing walls (34) of one or more vessels, and the body includes an outer surface aneurysmal region (43) configured to at least partially span one or more aneurysms of the one or more vessels; and
an anchoring layer (36) applied to the outer surface landing region and having anchors configured to anchor the endovascular implant to the one or more landing walls of the one or more vessels, and the outer surface aneurysmal region does not include the anchoring layer.

2. The endovascular implant of claim 1, wherein the anchoring layer includes an extracellular matrix layer (56).

3. The endovascular implant of claim 2, wherein the extracellular matrix layer is an artificial extracellular matrix layer.

4. The endovascular implant of claim 2, wherein the extracellular matrix layer is formed of an electrospun polymer material.

5. The endovascular implant of claim 1 further comprising an aneurysmal region layer applied to the outer surface aneurysmal region, the anchoring layer formed of a first material configured to promote a controlled wound response, the aneurysmal region layer formed of a second material configured to promote a controlled inflammatory reaction, and the first material is different than the second material.

6. The endovascular implant of claim 1, wherein the anchoring layer includes a prosthetic fabric (116) having first and second surfaces.

7. The endovascular implant of claim 6, wherein the prosthetic fabric includes first barbs (120) extending from the first surface.

8. The endovascular implant of claim 7, wherein the prosthetic fabric includes second barbs extending from the second surface.

9. The endovascular implant of claim 6 or 7, wherein the first or second barbs are the anchors.

10. The endovascular implant of claim 6 or 7, wherein the first and/or second barbs are formed from one or more bioresorbable polymers.

11. The endovascular implant of claim 6, 7, or 10, wherein the first and/or second barbs are coated with one or more natural biopolymers.

12. The endovascular implant of claim 1, wherein the anchoring layer includes a gripping polymeric material layer (150, 200).

13. The endovascular implant of claim 12, wherein the gripping polymeric material layer includes a base portion (152, 202) and protrusions (156, 208) extending from the base portion, the protrusions are the anchors.

14. The endovascular implant of claim 13, wherein the base portion includes first and second surfaces, the protrusions extending from the first surface, the second surface adhesively connected to the outer surface landing region.

15. The endovascular implant of claim 12 or 13, wherein the protrusions form a matrix of protrusions equally or randomly spaced from each other.
